# EUROPEAN PATENT APPLICATION

(11) **EP 1 364 645 A1**
(43) Date of publication of application: **26.11.2003**
(21) Application number: 03011214.8
(22) Date of filing: 16.05.2003
(51) Int. Cl.: A61K 31/19, A61K 38/36

(54) **Pharmaceutical combinations for topical use based on iron chelators, metalloprotease inhibitors and factor XIII for the treatment of lipodermatosclerosis and trophic lesions of the cutaneous tissues and muccous membranes**

(30) Priority: 21.05.2002 IT MI20021080
(71) Applicant: ZAMBONI, Paolo, 44100 Ferrara (IT); IZZO, Marcello, 80030 S.PAOLO BELSITO (NA) (IT)
(72) Inventor: Zamboni, Paolo, 44100 Ferrara (IT); Izzo, Marcello, 80030 S.Paolo Belsito (NA) (IT); Lanzara, Vincenzo, 44040 Chiesuol Del Fosso (FE) (IT)
(74) Representative: Minoja, Fabrizio, Dr.

(57) **Abstract**

The present invention relates to pharmaceutical combinations based on iron chelators, metalloprotease inhibitors and factor XIII for the topical treatment of lipodermatosclerosis and trophic lesions of the cutaneous tissues and mucous membranes in general, and venous ulcers in particular.

## Description

The present invention relates to pharmaceutical combinations based on iron chelators, metalloprotease inhibitors and factor XIII for the topical treatment of lipodermatosclerosis and trophic lesions of the cutaneous tissues and mucous membranes in general.

In particular, the invention relates to pharmaceutical combinations based on iron chelators, metalloprotease inhibitors and factor XIII for the topical treatment of venous ulcers.

The treatments currently available for trophic lesions of the cutaneous tissues and the mucous membranes in general, and venous ulcers in particular, which require the administration of numerous products for topical use such as carboxymethylcellulose in wafer, powder or granule form (Comfeel, Aquacel); hydrocolloids (Duoderm, Combiderm); multilayer absorbent dressings (Carboflex); calcium alginates (Sorbsan, Kaltostat); multilayer polyurethane dressings (Allevyn, Lyofoam); multilayer polyurethane dressings with sodium polyacrylate particles (Cutinova foam and cavity); multilayer polyurethane dressings with the addition of moisturisers and surfactants (PolyMen); hydrophilic polyurethane film dressings (Omniderm); cleansing/moisturising systems in water gel with propylene glycol (Intrasite gel); multilayer hypoallergenic hydrocolloid dressings (Biatain); dressings containing hyaluronic acid, either alone or associated with arginates (Transprocess, Trofoprocess, Hyalofill F or R, Jaloskin); heterologous animal collagen (Condress); fibrin glue (Tissucol); placenta extracts with growth factors (Placentex cream, ampoules etc.); substances containing antibiotics and aminoacids (Cicatrene powder, cream); micronised silver, either alone or associated with growth factors (Katoxyn powder, spray or Katogel); collagenase (Iruxol, Noruxol ointment); methylpyrrolidone chitosan, etc., are not wholly satisfactory, because they do not act in a complete, selective manner on the complex pathogenetic mechanisms of these disorders.

In venous ulcers, and especially in all lesions of the cutaneous tissues and mucous membranes with different etiologies, the collagen in the interstitial matrix breaks down through a common final route, namely the destroying enzymatic action performed by the metalloproteases. Metalloproteases 1,3,9 in particular are the most active in breaking down collagen fibers¹⁻². Moreover, in venous ulcers there is a constant interstitial iron overload which is highly damaging to the tissues when, as in this case, it is accompanied by a high concentration of free oxygen radicals due to the oxidative stress condition³⁻⁷.

In our laboratories, we have also observed that factor XIII is not present in the tissue of venous ulcers or other trophic lesions of the cutaneous tissues and mucous membranes. Moreover, it is not normally found in the cutaneous tissues of controls with no trophic lesions. This deficit is also observed when the individuals examined have normal expressions of factor XIII in the serum and platelets.

Factor XIII which, like its recombinants (particularly Val-34-Leu), belongs to the transglutamyltransferase family, repairs collagen broken down by the action of the metalloproteases in the tissues in which trophic lesions are found; in other words it cross-links the collagen molecules (but also fibronectin, vitronectin and other components of the extra-cellular matrix destroyed by the metalloproteases) broken by the metalloproteases, and therefore constitutes an important trophic tissue-growth factor because of its repair effect on broken-down collagen fibers, and probably because of its trophic activity on the fibroblasts. Unfortunately, factor XIII is inhibited by metalloproteases.

N-acetylcysteine (NAC) is a well-known mucolytic agent which has long been used to treat disorders of the respiratory apparatus. Various therapeutic uses of NAC for the treatment of skin diseases have also been described. For example, WO 95/00136 describes the use of NAC for the topical or systemic treatment of hyperkeratosis or disorders mediated by the metalloproteases, such as lichen planus, mouth ulcers and disorders involving blisters; US 4,708,965 describes the use of NAC in the treatment of ulcers and herpes rashes; WO 93/04669 describes the use of NAC in the treatment of wrinkles and atrophy of the skin; US 4,724,239 describes the use of NAC in the treatment of chemical ulcers; and finally, US 6,166,084 describes the use of NAC in the treatment of chronic sores such as venous and diabetic ulcers and bedsores.

It has now been discovered that the topical use of drugs able to inhibit the metalloproteases and chelate interstitial iron, associated with factor XIII, provides an effective treatment not only for venous ulcers, but also for the immediately preceding clinical stage, namely clinical stage CEAP C4 (lipodermatosclerosis), in which identical, though less serious alterations take place.

The present invention provides pharmaceutical combinations based on:
a) iron chelators, and/or
b) metalloprotease inhibitors, and
c) factor XIII.

They perform a synergic action with a very high level of therapeutic activity, which makes them useful in the topical treatment of trophic lesions of the integuments and mucous membranes, especially venous ulcers.

Natural or recombinant factor VIII can be used.

The term "iron chelators" refers in particular to compounds selected from desferroxamine, hydroxyamates, catecholates, aminocarboxylates, α-ketohydroxypyridines, bidentate iron chelators, arginine thiazolidine carboxylate, glutathione (TAD), thioctic (lipoic) acid, thiopronine, flavonoids, ferrozine and pyridoxal fluorobenzoyl hydrazone (PFBH).

Of the hydroxyamates, cholylhydroxamic acid and salicylhydroxamic acid are particularly preferred. Of the catecholates, 2,2-dihydroxybenzoic acid and 2,3-dihydroxybenzoic acid are particularly preferred. Of the aminocarboxylates, ethylenediaminetetraacetic acid and diethylenetriaminepentaacetic acid are particularly preferred. Of the bidentate iron chelators, deferiprone and L1 are particularly preferred. Of the flavonoids, quercetin, catechin, kaempferol and hesperidin are particularly preferred.

The term "metalloprotease inhibitors" refers in particular to compounds selected from Barimastat, Marimastat, Prinomastat, AG3340 and Bay219566.

According to a particularly preferred aspect of the invention, N-acetylcysteine will be present both as iron chelator and metalloprotease inhibitor. According to a further preferred aspect of the invention, lipoic acid will be present both as iron chelator and metalloprotease inhibitor. As a result, combinations according to the invention may include NAC or lipoic acid and factor XIII, and possibly other iron chelators and/or metalloprotease inhibitors.

According to the invention, the iron chelators will be present in amounts ranging between 10 and 50%, the metalloprotease inhibitors in amounts ranging between 10 and 50%, and factor XIII in amounts ranging between 10 and 50%.

According to a preferred aspect, when N-acetylcysteine (or lipoic acid) is present both as iron chelator and metalloprotease inhibitor, its concentration may range between 10 and 50%.

The pharmaceutical combinations according to the invention will be formulated in the form of creams, ointments, gels, sprays, emulsions, and advanced dressing systems with transdermal release of the active constituent. Advanced dressing systems which allow release of the active constituents onto the lesion for several days and simultaneous application of therapeutic elastocompression outside it will be particularly preferred, to allow more convenient, active management of the disorder, as dressings and bandages no longer need to be changed one or more times a day but every 7-10 days, which is more suitable for public ulcer-care services.

The principles of disinfection and debridment and the collateral medical, surgical and conservative treatment currently used will remain unchanged.

In the case of cream, gel, emulsion, ointment, paste, spray or liquid formulations of the combinations according to the invention, the dose can vary from one to three daily applications, possibly administered by patients themselves; in the case of delivery by means of advanced dressing systems, the dressing could be changed after 2-10 days.

An example of a formulation according to the invention is set out below.

### Example

| | |
|---|---|
| Cream containing | |
| NAC | 20% |
| factor XIII | 20% |
| Propylene glycol q.s. for | 100 ml |
| Other excipients q.s. for | 100 ml. |

The combinations according to the invention has been experimentally tested. The results are set out below.

45 patients suffering from "pure" venous ulcers underwent randomised treatment: 22 were treated with conventional dressings and 23 by adding a topical application of a pharmaceutical form of N-acetylcysteine. 10 of the latter patients were given the substance in undiluted form, 8 patients received it diluted to 50% with saline, and the other 5 patients were given a 20% cream based on said active constituent. The lesion tissue repair rate was measured in the two groups by scanning the edge of the lesion. The area was processed by software able to measure the areas of irregular polygons. The serial measurements of the lesions, divided by the healing time, gave the tissue repair rate in mm²/day. The repair rate was 1.86 mm²/day for the control group, and 2.66 (p<0.02) for the treated group.

These *in vivo* data demonstrate that in the case of venous ulcers, local iron chelation and metalloprotease inhibition shorten the healing time.

These results consequently demonstrate that the said molecules repair damage to the collagen by the metalloproteases, and that their topical use reduces and accelerates lesion healing time.

### REFERENCES

1. Herouy Y, Trefzer D, Zimpfer U, Schopf E, Wanscheidt W, Norgauer J. Matrix metalloproteinases and venous leg ulceration. Eur J Dermatol. 2000 Apr-May; 10(3):173-80.
2. Herouy Y.: Matrix metalloproteinases in skin pathology. Int J Mol Med. 2001 Jan; 7(1):3-12.
3. Weir MP, et al.: Haemosiderin and tissue damage. Cell Biochem Funct. 1984 Oct; 2(4):186-94.
4. O'Connell M, et al.: Formation of hydroxyl radicals in the presence of ferritin and haemosiderin. Is haemosiderin formation a biological protective mechanism? Biochem J. 1986 Mar 15; 234(3).
5. Ackerman Z, Seidenbaum M, Loewenthal E, Rubinow A. Overload of iron in the skin of patients with varicose ulcers. Possible contributing role of iron accumulation in progression of the disease. Arch Dermatol 1988; 124:1376-1378.
6. Ward RJ, Legssyer R, Henry C, Crichton RR: Does the haemosiderin iron core determine its potential for chelation and the development of iron-induced tissue damage? Eur J Biochem 1994 Oct 1; 225(1):187-94.
7. Weir MP, et al. Haemosiderin and tissue damage. Cell Biochem Funct. 1984 Oct; 2(4):186-94. Review.
8. O'Connell MJ, et al. Ferritin and haemosiderin in free radical generation, lipid peroxidation and protein damage. Chem Phys Lipids. 1987 Nov-Dec; 45(2-4):241-9. Review.

## Claims

1. Pharmaceutical combinations based on:
a) iron chelators, and/or
b) metalloprotease inhibitors, and
c) factor XIII,
for the topical treatment of trophic lesions of the cutaneous tissues and mucous membranes.

2. Pharmaceutical combinations as claimed in claim 1, wherein the iron chelator and metalloprotease inhibitor is N-acetylcysteine.

3. Pharmaceutical combinations as claimed in claim 1, wherein the iron chelators are present in the amount of 10 to 50% of the weight of the composition.

4. Pharmaceutical combinations as claimed in claim 1, wherein the metalloprotease inhibitors are present in the amount of 10 to 50% of the weight of the composition.

5. Pharmaceutical combinations as claimed in claim 1, wherein factor XIII is present in the amount of 10 to 50% of the weight of the composition.

6. Pharmaceutical combinations as claimed in claims 1 and 2, wherein N-acetylcysteine is present in the amount of 10 to 50% of the weight of the composition.

7. Pharmaceutical combinations as claimed in claim 1, wherein the iron chelators are selected from desferroxamine, hydroxyamates, catecholates, aminocarboxylates, α-ketohydroxypyridines, bidentate iron chelators, arginine thiazolidine carboxylate, glutathione (TAD), thioctic (lipoic) acid, thiopronine, flavonoids, ferrozine and pyridoxal fluorobenzoyl hydrazone (PFBH).

8. Pharmaceutical combinations as claimed in claim 7, wherein the hydroxyamates are selected from cholylhydroxamic acid and salicylhydroxamic acid.

9. Pharmaceutical combinations as claimed in claim 7, wherein the catecholates are selected from 2,2-dihydroxybenzoic acid and 2,3-dihydroxybenzoic acid.

10. Pharmaceutical combinations as claimed in claim 7, wherein the aminocarboxylates are selected from ethylenediaminetetraacetic acid and diethylenetriaminepentaacetic acid.

11. Pharmaceutical combinations as claimed in claim 7, wherein the bidentate chelators are selected from deferiprone and L 1.

12. Pharmaceutical combinations as claimed in claim 7, wherein the flavonoids are selected from quercetin, catechin, kaempferol and hesperidin.

13. Pharmaceutical combinations as claimed in claim 1, wherein the metalloprotease inhibitors are selected from Barimastat, Marimastat, Prinomastat, AG3340 and Bay219566.

14. Pharmaceutical combinations as claimed in the preceding claims, in the form of creams, ointments, gels, sprays, emulsions, pastes, powders, granules, transdermal or occlusive systems, or in liquid form.
